# EUROPEAN PATENT SPECIFICATION

(11) **EP 1 400 584 B1**
(45) Date of publication and mention of the grant of the patent: **18.04.2007**
(21) Application number: 03021093.4
(22) Date of filing: 18.09.2003
(51) Int. Cl.: C12M 3/04, B65D 21/02

(54) **Roller Bottle**
Rollflasche
Bouteilles tournantes

(30) Priority: 20.09.2002 US 412203 P
(43) Date of publication of application: 24.03.2004
(73) Proprietor: Becton Dickinson and Company, Franklin Lakes, NJ 07417-1880 (US)
(72) Inventor: Whitley, Kenneth W., Raleigh, North Carolina 27615 (US)
(74) Representative: Weber, Thomas

(56) References cited:
- EP-A- 0 614 967
- US-A- 4 810 652
- PATENT ABSTRACTS OF JAPAN vol. 2002, no. 08, 5 August 2002 (2002-08-05) & JP 2002 114225 A (ISHIZUKA GLASS CO LTD), 16 April 2002 (2002-04-16)

## Description

### BACKGROUND OF THE INVENTION

### 1. Field of the Invention

This invention relates to a container for cell culture production and more particularly to a roller bottle having a recessed portion at its bottom end for accommodating an adjacently stacked roller bottle in a manner which allows gases to enter a gas-permeable cap of the stacked bottles.

### 2. Description of Related Art

One type of container commonly used in the laboratory for culturing of cells is known as a "roller bottle". Roller bottles are generally cylindrically shaped and are adapted to rotate about their axes. The internal surfaces of such roller bottles are for providing active surfaces for cells. A liquid growth medium is introduced into the roller bottle. The rotating movement of the bottle keeps the internal surfaces wetted with a liquid medium, thereby encouraging the growth of cells. Rotating rollers of an appropriate apparatus are employed to rotate these roller bottles. The roller bottles are typically arranged on the rollers of the apparatus in end-to-end, stacked relationship, with one end of one bottle being abutted against the opposite end of an adjacent bottle. EP 0 614 967 A describes such roller bottles.

It is desirable to grow large amounts of cells, mostly for cell by-products, such as pharmaceutical substances that are secreted by cells. The standard roller bottles have been successful in increasing the yield of cell growth insofar as the entire inside peripheral surface area can be utilized for cell culturing. In general, cell yields can be increased by maintaining ideal conditions for cell growth.

In typical culture systems, pH is maintained near physiologic levels by utilizing a buffering system in the tissue culture fluid, in conjunction with an incubator in which carbon dioxide (CO₂) is infused at a rate sufficient to maintain a concentration in the incubator atmosphere of approximately 5-7 volume percent. The CO₂ reacts with water to form a weak acid and a carbonic acid, which in turn inter-reacts with the buffering system to maintain the pH near physiologic levels. Entry of CO₂ from the incubator into the tissue culture vessel is generally achieved by utilizing a closure on the vessel such as, a loose fitting cap, a stopper or a cap with a permeable membrane. Equilibration in the vessel is maintained by allowing gas exchange with the inside of the vessel and the atmosphere of the incubator while preserving sterility and preventing liquid leakage.

One of the problems associated with roller bottles provided with caps including gas-permeable membranes has been that the cap can seal against the bottom of an adjacently stacked bottle when the two are stacked end-to-end. This can result in a gas-tight seal which prevents gases from flowing in and out of the permeable membrane, discouraging cell growth within the bottle.

A need exists, therefore, for an improved roller bottle having a means for allowing gases to exchange with the inside of a similar roller bottle with which it is adjacently stacked. By preventing a gas-tight seal from forming between the two stacked roller bottles, the cell cultures would be prevented from exposure to undesirable changes in the pH of the system.

### SUMMARY OF THE INVENTION

The present invention provides a container for cell growth culturing, such as a roller bottle. The container includes an elongate cylindrical wall having a closed bottom end and an opposed projecting neck portion defining a liquid opening. The closed bottom end includes an inwardly directed recessed portion including a planar surface. The recessed portion accommodates a neck portion of an adjacently stacked similar container. The planar surface includes a plurality of ribs extending therefrom so as to space the neck portion of the similar container from the planar wall.

The invention further provides a container assembly including the container just described; and a venting cap including a gas-permeable membrane for closing the liquid opening.

The invention solves a need in the art by providing ribs at the bottom of the container which prevent the bottom of the bottle from sealing against the top of a similar bottle when the two are stacked end-to-end.

### BRIEF DESCRIPTION OF THE DRAWINGS

FIG 1 is a longitudinal cross-section of a roller bottle of the invention.

FIG 1A is a partial sectional view of the neck portion of the roller bottle of FIG 1.

FIG 1B is a partial sectional view of a cap for closure of the liquid opening of the roller bottle of FIG 1.

FIG 2 encompasses bottom planar and side elevation views of the roller bottle of FIG 1.

FIG 3 is a cross-section of the roller bottle of FIG 1 in stacked relationship with a similar bottle including a gas-permeable cap.

### DETAILED DESCRIPTION OF THE INVENTION

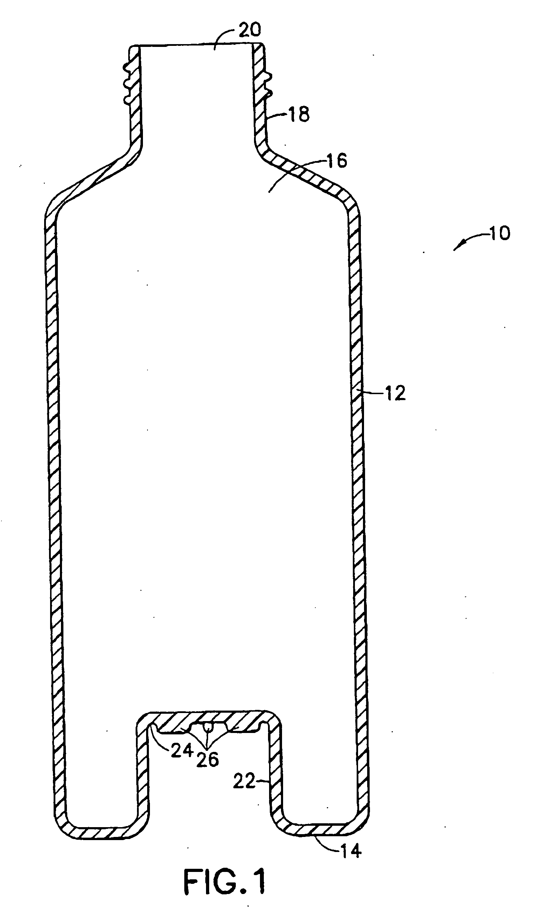

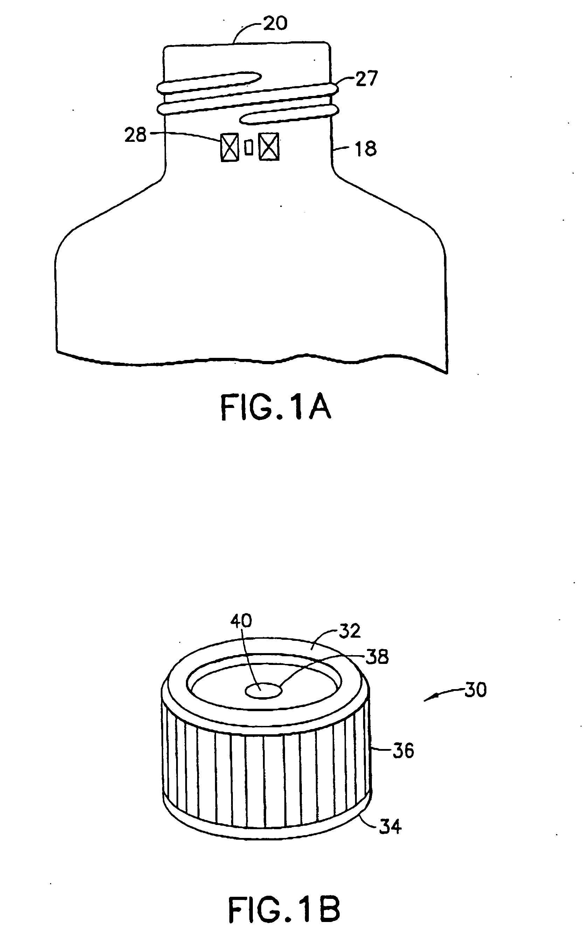

Referring now to the drawings in which like reference characters refer to like parts throughout, FIG 1 shows a container for cell growth culturing in accordance with the present invention. In particular, FIG 1 shows roller bottle 10. As can be seen in FIG 1, roller bottle **10** includes a cylindrical wall **12** which extends from closed bottom end **14** to a top **16**. Extending from top **16**, and integral therewith, is a projecting neck portion **18** defining a liquid opening **20** at the end opposite the closed end **14**. Neck **18** is integral with the bottle **10** and defines a cylindrical conduit having one end integral with the container and the other end defining an opening through which the cells and culture fluids may be introduced into the body of the container. Closed end **14** of bottle **10** includes an inwardly directed recessed portion **22** which is generally frustoconical in shape. The frustoconical portion is shaped and proportioned to correspond to a projecting neck of a similar second bottle so as to accommodate the neck of the second bottle when the two are adjacently stacked end-to-end.

With reference now to FIG 1A, it can be seen that the neck portion **18** of the roller bottle **10** of FIG 1 may include external screw threads **27** for receiving an internally screw threaded cap thereon as will be described below. It is anticipated that other cap connections such as bayonette connections may also be used. It is noted that neck **18** may include a locking arrangement **28** for holding a cap in a locked open position on the roller bottle for maintaining the roller bottle open to the environment surrounding it.

As shown in FIG 1B, a roller bottle according to the present invention may be provided with a cap **30** for elongate neck portion **18**. Cap **30** has a top surface **32** and an annular outer skirt **36** extending from the top surface to a bottom stop ledge **34.** Cap **30** further has a central orifice **38** extending through with top surface **32.** A gas-permeable membrane **40** is affixed to the interior of surface **32** to close orifice **38**.

The gas-permeable membrane **40** may be made from any suitable gas-permeable material so long as it provides free passage of gases such as oxygen and carbon dioxide into the hollow chamber defined by the cylindrical wall of the container of the present invention, while preventing bacteria and fungi from passing therethrough. Membrane materials provide adequate rates of carbon dioxide and oxygen permeability while preventing passage of microorganisms. Suitable gas-permeable materials include polyethylene, polycarbonate, acrylic co-polymers and polytetrafluoroethylene.

A problem associated with prior art roller bottles has been the tendency for the planar wall **24** of the recessed portion **22** at the bottom end **14** to seal up against the neck portion **18** of a similar bottle when the two are adjacently stacked. In situations where a vented cap is employed, it is necessary to prevent such a seal so as to allow rapid and uniform equilibration between the gases in the incubator and the bottle, encouraging cell growth within the bottle. In the presence of a seal between the bottom of a bottle and the top of another bottle, gases would not be allowed to flow in and out of the bottle. This may lead to low cell yield, and likely cell death due to the absence of a controlled cell culture environment. For example, as described above, in the absence of an adequate infusion of carbon dioxide into the bottle, the pH of the culture system will not be maintained near desired physiologic levels.

The present invention solves a need in the art by contracting the recessed portion **22** to have a plurality of projecting ribs **26** on a planar surface **24.** Ribs **26** are integral with planar surface **24** and extend therefrom so as to allow spacing of the neck portion **18** of a similar bottle from planar wall **24**. Ribs **26** prevent the planar surface from sealing against the top of another bottle when the two are adjacently stacked. The ribs may vary in shape, as well as the extent to which they project from planar surface **24**. In general, the more the ribs project from the planar surface, the greater the space created between the neck portion of the adjacently stacked similar bottle and the planar wall of the recessed portion of the container of the invention.

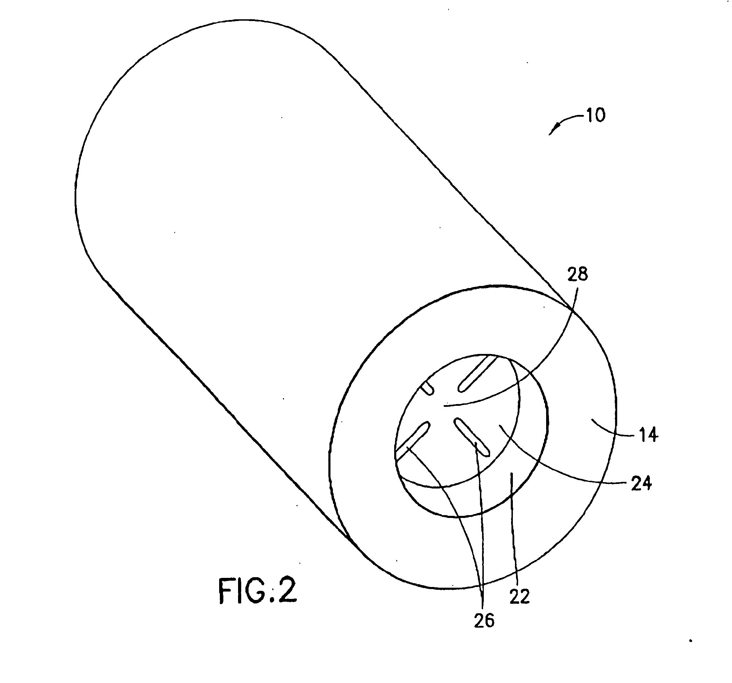

Referring now to FIG 2, one embodiment is shown wherein the ribs **26** projecting from planar surface **24** extend from a point **28** proximal to the longitudinal axis of the container of the present invention toward recess wall **22** where they terminate, the ribs being about equally spaced about the longitudinal axis of the container.

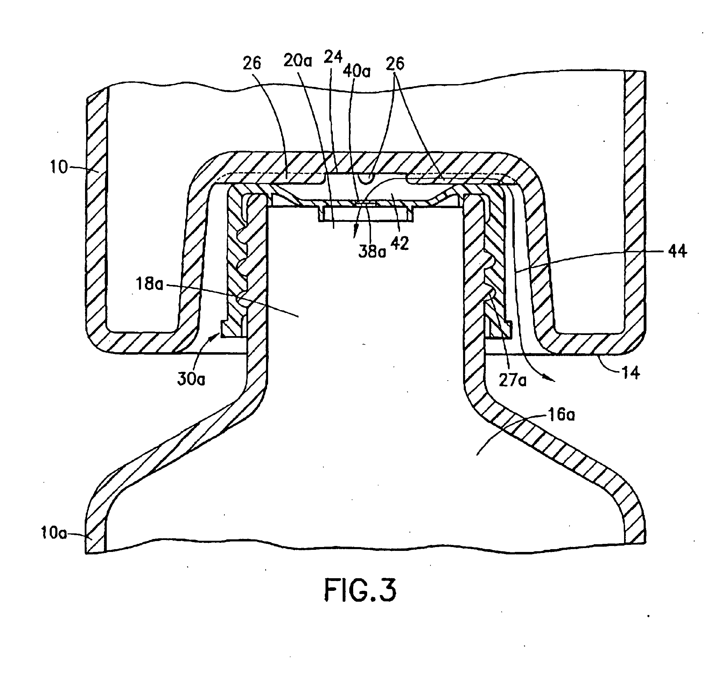

As shown in FIG 3, when one container **10** is stacked on a similar container **10a**, the ribs **26** of bottle **10** extending from planar surface **24** create a space between surface **24** of bottle **10** and neck portion **18a** of bottle **10a.** In particular, cap **30a** of bottle **10a** rests against ribs **26** of bottle **10.** This allows sufficient space **42** to be created between the top of cap **30a** of bottle **10a** and surface **24** of bottle **10** to allow gases **44** to flow in and out of orifice **38a** of cap **30a.** In one embodiment, cap **30a** is provided with gas-permeable membrane **40a** which allows rapid equilibration of gases between the inside and outside of bottle **10a**.

Whereas the container of the present invention is shown in the drawings as having a smooth cylindrical wall, it is well within the contemplation of the present invention that the container may have pleated or corrugated surfaces extending longitudinally or axially about the roller bottle. For example, it is known that pleats formed in the walls of the roller bottle increase the effective surface area for cell growth.

The container of the present invention may be produced as a single one-piece or unitary structure by a simple blow-molding technique. Because of this, the roller bottle of the invention may be mass-produced inexpensively. Moreover, the roller bottle of the invention is configured so that it may be used in a conventional laboratory roller bottle apparatus.

In viewing the conditions for producing roller bottles in accordance with the invention, a variety of thermoplastic materials may be utilized, including, for example, polystyrene, polyethylene terephthalate, the polyolefins and polyvinyl chloride. Polystyrene is particularly desirable because it has been found that cells appear to grow better and in greater numbers on this material.

With the construction of the container of the present invention described herein, stacking with containers of like size and shape is possible. In particular, as shown in FIG 3, containers such as roller bottles according to the present invention may be arranged adjacently as shown in FIG 3 so that there is no lost space between adjoining containers, while at the same time eliminating the possibility of forming an air-tight seal between the planar wall of the recess of the bottom of one inventive container and the neck portion of a similar second container.

## Claims

1. A container for cell growth culturing comprising:
an elongate cylindrical wall having a closed bottom end and an opposed projecting neck portion end defining a liquid opening, said closed bottom end including an inwardly directed recessed portion for accommodating a neck portion end of an adjacent stacked similar container, **characterised in that** said recessed portion includes a planar surface having at least one rib extending therefrom for defining a space between said neck portion of said similar container and said planar surface.

2. The container of claim 1, wherein said space permits gases to enter into and out of a liquid opening of said adjacent stacked similar container.

3. The container of claim 1, wherein said container is a stackable roller bottle.

4. The container of claim 1, wherein said rib is integral with said planar surface.

5. The container of claim 1, wherein said recessed portion further includes a side wall.

6. The container of claim 5, wherein said rib radiates from a point proximal to the longitudinal axis of the container toward said side wall of said recessed portion where said rib terminates.

7. The container of claim 6, wherein a plurality of said ribs is provided, and said ribs are about equally spaced about the longitudinal axis of the container.

8. The container of claim 1, wherein said recessed portion is generally frustoconical in shape.

9. A method of stacking containers for cell growth culturing comprising:
providing a first container having a closed bottom end and an opposed projecting neck portion end defining a liquid opening, said closed bottom end including an inwardly directed recessed portion, said recessed portion including a planar surface having at least one rib extending therefrom;
providing a second container; and
stacking said first container and said second container with said neck portion of said second container being nested in said recessed portion of said first container, wherein said rib spaces said neck portion of said second container from said planar surface of said first container.

## Patentansprüche

1. Behälter für das Zellenwachstum, mit:
einer länglichen zylindrischen Wand mit einem geschlossenen unteren Ende und einem entgegengesetzten vorstehenden Halsteilende, das eine Flüssigkeitsöffnung bildet, wobei das geschlossene untere Ende einen nach innen gerichteten Ausnehmungsteil zum Aufnehmen eines Halsteilendes eines benachbarten gestapelten im Wesentlichen gleichen Behälters aufweist,
**dadurch gekennzeichnet, dass**
der Ausnehmungsteil eine planare Fläche mit mindestens einer davon vorstehenden Rippe aufweist, die einen Zwischenraum zwischen dem Halsteil des im Wesentlichen gleichen Behälters und der planaren Fläche verursacht.

2. Behälter nach Anspruch 1, bei dem es der Zwischenraum ermöglicht, dass Gase in eine Flüssigkeitsöffnung des benachbarten gestapelten im Wesentlichen gleichen Behälters eintreten und aus dieser austreten.

3. Behälter nach Anspruch 1, wobei der Behälter eine stapelbare Rollflasche ist.

4. Behälter nach Anspruch 1, bei dem die Rippe einstückig mit der planaren Fläche ausgebildet ist.

5. Behälter nach Anspruch 1, bei dem der Ausnehmungsteil ferner eine Seitenwand aufweist.

6. Behälter nach Anspruch 5, bei dem die Rippe von einer proximal zu der Längsachse des Behälters liegenden Stelle zu der Seitenwand des Ausnehmungsteil, an der die Rippe endet, verläuft.

7. Behälter nach Anspruch 6, bei dem mehrere Rippen vorgesehen sind, die in ungefähr gleichem Abstand um die Längsachse des Behälters angeordnet sind.

8. Behälter nach Anspruch 1, bei dem der Ausnehmungsteil im Wesentlichen kegelstumpfförmig ausgebildet ist.

9. Verfahren zum Stapeln von Behältern für das Zellenwachstum, mit folgenden Schritten:
Bereitstellen eines ersten Behälters mit einem geschlossenen unteren Ende und einem entgegengesetzten vorstehenden Halsteilende, das eine Flüssigkeitsöffnung bildet, wobei das geschlossene untere Ende einen nach innen verlaufenden Ausnehmungsteil aufweist, der eine planare Fläche mit mindestens einer von dieser vorstehenden Rippe aufweist;
Bereitstellen eines zweiten Behälters; und
Stapeln des ersten Behälters und des zweiten Behälters, wobei der Halsteil des zweiten Behälters in den Ausnehmungsteil des ersten Behälters eingefügt wird, wobei die Rippe den Halsteil des zweiten Behälters von der planaren Fläche des ersten Behälters beabstandet.

## Revendications

1. Récipient pour la culture cellulaire comprenant :
une paroi cylindrique allongée présentant une extrémité inférieure fermée et une extrémité de partie de col en saillie opposée définissant une ouverture pour le liquide, ladite extrémité inférieure fermée comportant une partie évidée dirigée vers l'intérieur destinée à loger une extrémité de partie de col d'un récipient similaire superposé et adjacent, **caractérisé en ce que** ladite partie évidée comporte une surface plane présentant au moins une nervure s'étendant depuis celle-ci pour définir un espace entre ladite partie de col dudit récipient similaire et ladite surface plane.

2. Récipient selon la revendication 1, dans lequel ledit espace permet aux gaz de pénétrer à l'intérieur d'une ouverture pour le liquide dudit récipient similaire superposé et adjacent et d'en sortir.

3. Récipient selon la revendication 1, dans lequel ledit récipient est un flacon roulant superposable.

4. Récipient selon la revendication 1, dans lequel ladite nervure est formée d'un seul tenant avec ladite surface plane.

5. Récipient selon la revendication 1, dans lequel ladite partie évidée comporte en outre une paroi latérale.

6. Récipient selon la revendication 5, dans lequel ladite nervure rayonne d'un point proximal par rapport à l'axe longitudinal du récipient en direction de ladite paroi latérale de ladite partie évidée où se termine ladite nervure.

7. Récipient selon la revendication 6, dans lequel une pluralité desdites nervures sont fournies, et lesdites nervures sont également placées à équidistance autour de l'axe longitudinal du récipient.

8. Récipient selon la revendication 1, dans lequel ladite partie évidée présente une forme généralement frustoconique.

9. Procédé de superposition de récipients pour la culture cellulaire comprenant :
la fourniture d'un premier récipient présentant une extrémité inférieure fermée et une extrémité de partie de col en saillie opposée définissant une ouverture pour le liquide, ladite extrémité inférieure fermée comportant une partie évidée dirigée vers l'intérieur, ladite partie évidée comportant une surface plane présentant au moins une nervure s'étendant depuis celle-ci ;
la fourniture d'un second récipient ; et
la superposition dudit premier récipient et dudit second récipient avec ladite partie de col dudit second récipient emboîtée dans ladite partie évidée dudit premier récipient, dans lequel ladite nervure espace ladite partie de col dudit second récipient de ladite surface plane dudit premier récipient.
